# EUROPEAN PATENT SPECIFICATION

(11) **EP 4 123 663 B1**
(45) Date of publication and mention of the grant of the patent: **17.12.2025**
(21) Application number: 21187232.0
(22) Date of filing: 22.07.2021
(51) Int. Cl.: G16H 40/67, G16H 20/40, G06F 3/14, G06F 3/04886, G06F 3/0488, A61G 13/06, A61G 13/08

(54) **A MEDICAL SYSTEM WITH AN OPERATING TABLE AND A REMOTE CONTROL, AND A METHOD OF OPERATING A MEDICAL SYSTEM WITH AN OPERATING TABLE**
MEDIZINISCHES SYSTEM MIT OPERATIONSTISCH UND FERNBEDIENUNG, SOWIE EIN VERFAHREN ZUM BETREIBEN EINES MEDIZINISCHEN SYSTEMS MIT EINEM OPERATIONSTISCH
SYSTÈME MÉDICAL COMPRENANT UNE TABLE D'OPÉRATION ET UNE TÉLÉCOMMANDE ET PROCÉDÉ DE FONCTIONNEMENT D'UN SYSTÈME MÉDICAL COMPRENANT UNE TABLE D'OPÉRATION

(43) Date of publication of application: 25.01.2023
(73) Proprietor: Maquet GmbH, 76437 Rastatt (DE)
(72) Inventor: GOLDE, Tim, 76356 Weingarten (DE); ERTEL, Philipp, 76646 Bruchsal (DE); KIEFFER, Cédric, 67100 Strasburg (FR); WELSCH, Michael, 76571 Gaggenau (DE)
(74) Representative: Zacco GmbH

(56) References cited:
- EP-A2- 3 851 065
- WO-A1-2014/043659
- US-A1- 2014 297 897
- US-A1- 2016 140 307

## Description

The present disclosure relates to a medical system with an operating table and a remote control, an operating table, a remote control, and a method for operating a medical system with an operating table.

Medical devices, such as operating tables, can be controlled remotely by use of a remote control in a convenient way. Compared to remote controls without touchscreens, remote controls with touchscreens can provide more information and flexibility to the user for interacting with the medical device.

A remote control for controlling a medical appliance is described in WO 2016/131659 A1.

US2016140307 discloses a medical system includes a medical apparatus, a computer, a user input device, and at least one feature in communication with and controlled by the computer. The computer is in communication with the user input device, which is configured and arranged to allow a user to purchase the use of the feature. The computer is configured to enable the use of the feature after the user purchases use of the feature.

Currently an operating table remote control and the associated operating table can be regarded as two independent systems, which interact with each other via a command and information exchange interface. The operating table is responsible for carrying out all movements of the table, interfacing with other systems in a hospital, interpreting commands received from the remote control, and generating commands and information to send to the remote control. The remote control system is responsible for accepting user input over hardware buttons and touchscreen, if any, for generating a graphical user interface (GUI) shown to the user on a screen or touchscreen of the remote control, for interpreting commands and information it receives from the table, and for sending user generated commands to the table.

As the operating table and the remote control are independent systems, all capabilities provided for interacting with the table via the remote control to a user must be included in the software for both systems. This also applies to state of the art remote controls used in conjunction with other medical devices that are not operating tables.

It is therefore an object of the present invention to provide an improved medical system with a medical device, in particular an operational table and a remote control. It is also an object of the invention to provide an improved medical device and an improved remote control. Moreover, it is an object of the present invention to provide an improved method of operating a medical system with a medical device and a remote control. The medical devices may be, for example, an operating table for supporting a patient.

At least in some embodiments of the present invention, a medical system in accordance with the present invention comprises:
a medical device, in particular an operating table, having at least one computer system;
a remote control for remotely controlling operation of the medical device, the remote control comprising a screen;
the computer system and the remote control being configured to communicate with each other via a communication interface;
the computer system being configured to provide via the communication interface a graphical user interface (GUI) to the remote control, and the remote control being configured to execute the graphical user interface; and
the computer system, optionally, being configured to control at least a portion of the screen by the graphical user interface, when executed in the remote control, and, further optionally, to initiate the display of at least one graphical user interface element on the screen of the remote control, in particular in the portion of the screen.

Thus, at least in some embodiments, the display of the remote control can be controlled by a graphical user interface of the computer system, and hence, the remote control, or at least its display, can be regarded as an extension of the computer system. For example, the remote control can serve to display image data provided by the computer system to the remote control via the communication interface, and to provide user input directly to the computer system by use of the graphical user interface, while the remote control itself neither has to interpret any received data nor user input.

As least in some embodiments, the computer system and the remote control can be implemented as a client-server system, in which the display of the client, which corresponds to the remote control, hosts the GUI of the server system, which corresponds to the computer system, for example a computer within an operating table. The remote control can therefore be a means for displaying the at least one graphical user interface element, but a separate GUI system of the remote control, if any, does not obtain any information about the provided data, except for what is necessary to display it. In some embodiments, the remote does not need to be able to recognize the data in order to associate it with predetermined displaying instructions in the remote control (such as how to convert the information into human readable form, and how fit it into a predetermined layout), instead the information along with basic displaying instructions is enough that the remote does not need to recognize data and associate it with any predetermined displaying instructions.

In some embodiments, the at least one graphical user interface element is at least one of the following: an image, an input field, such as a slider or a tick box. The graphical user interface element can be any element that can be shown on a display. For example, it can be an image or a sequence of images. However, preferably, live images, for example of a patient are not provided. The computer system can determine the way how the visualized elements are displayed to the user. For example, it can determine at least one of the following: font, colour, location, making user readable, contextual information. This is in particular due to the fact that the computer system provides the GUI elements.

In some embodiments, the at least one graphical user interface element is at least one of the following:
an input control element, such as a checkbox, a radio button, a dropdown list, a list box, a button, a dropdown button, a toggle, a date field, an alphabetic keyboard, a numeric keyboard, an icon as a button, an interactive image;
a navigational component, such as a search field, a slider, an icon;
an informational component, such as a tooltip, an icon, a progress bar, a notification, a message box, a modal window, an image, a formatted text.

Radio buttons are used to allow users to select one item at a time. A dropdown button consists of a button that when clicked displays a drop-down list of mutually exclusive items. A toggle button allows the user to change a setting between two states. They are most effective when the on/off states are visually distinct. A slider, also known as a track bar, allows users to set or adjust a value. An icon is a simplified image serving as an intuitive symbol that is used to help users to navigate the system. An icon can be hyperlinked.

In some embodiments, the at least one graphical user interface element corresponds to at least one image and the computer system is configured to provide or generate data that includes image data of the at least one image. The computer system can be configured to send the data to the remote control for display of the at least one image on the screen. The image can be displayed on the screen, in particular in the portion, which is controlled by the graphical user interface. The remote control is not required to interpret the image or generate the image on its own. Thus, the complexity that has to be put into the remote control can be reduced, while the functionality and user experience of the remote control can still be enhanced due to using the graphical user interface of the computer system which serves to control the screen of the remote control.

In some embodiments, the data further includes displaying instructions for displaying the at least one image on the screen; and the remote control is configured to display, in accordance with the displaying instructions, the at least one image on the screen. The displaying instructions can provide information, such as an anchor point, that defines where on the screen the image should be displayed.

In some embodiment, the graphical user interface element corresponds to human readable and/or human ready instructions or information and the computer system is configured to provide data that includes the instructions or information to the remote control. The instructions can be basic instructions, such as text instructions, and the information can include text, symbols, and numbers. The remote control can be configured to carry out the instructions. For example, the display of some elements on the screen could be initiated by instructions, as opposed to directly providing images. For instance, a list of WLAN networks in the vicinity of the medical device could be sent as data which includes a set of text items and instructions where to display them. An "OK" and a "cancel" button could be created simply by sending data comprising instructions to show a "Cancel" button at the top left and an "OK" button at the top right of the screen. Moreover, information can be displayed on the screen.

In some embodiments, the instructions include an identifier for at least one image and displaying information where to display the at least one image on the screen, wherein the remote control is configured to identify, based on the identifier, the at least one image in a database on the remote control and to display the at least one image on the screen in accordance with the displaying information. For example, an "OK" and a "cancel" button could be created simply by sending data comprising instructions to show a "Cancel" button at the top left and an "OK" button at the top right of the screen. Images of the "OK" and a "cancel" button can be stored on a local database of the remote control.

At least in some embodiments, the computer system can control remotely the remote control and in particular its screen by use of Virtual Network Computing (VNC), which is a graphical desktop-sharing system. The VNC can be used to share the computer system's graphical user interface with the remote control. Thus, the computer system can, based on VNC and by using the Remote Frame Buffer protocol (RFB), remotely control the remote control, either fully or partially. The remote control can transmit keyboard and/or touchscreen events to the computer system, which can send graphical-screen updates to the remote control.

In some embodiments, the computer system is configured to receive user input from the remote control, and to initiate, in accordance with the received user input, the display of at least one updated graphical user element. The updated graphical user element can for example include updated image data, which is provided or generated by the computer system in accordance with the received user input. The computer system can transmit the updated image data to the remote control. User input, for example by pressing a key or a button on the remote control, is communicated to the computer system and further processed there. The user input can be related to the at least one image shown on the display, and the user input can be aimed at causing a change of a setting of the medical device.

In some embodiments, the computer system can be configured, in particular by use of a further computer system that controls the operation of the medical device, to cause a change of a setting of the medical device in accordance with received user input. Moreover, the updated image data can reflect, for example, a corresponding change of the settings and comprise at least one updated image, which shows the medical device in the new setting. The updated image data can be sent to the remote control, in particular in form of updated data comprising the updated image data of at least one updated image and displaying instructions for displaying the at least one updated image on the screen. In some embodiments, the computer system is part of an operating table and causes one or more sections of the operating table to move in response to the received user input from the remote. In some embodiments, the computer then updates an image of the operating table to reflect the movement of the operating table, and then sends the updated image of the operating table to the remote control where it is displayed.

In some embodiments, the screen is a touch screen and the computer system is configured to receive positional information of a user input on the touchscreen and to initiate the display of at least one updated graphical user interface element on the screen in dependence on the received positional information of the user input. For example, the computer system can generate or provide updated image data based on the received positional information of the user input. For example, the positional information of a user input can relate to coordinates on the touchscreen of a touch event that is detected. The coordinates of the touch event can be sent from the remote control to the computer system, where the coordinates are interpreted. In some embodiments, an updated image of the medical device can be generated in dependence on the coordinates. For example, the medical device can be an operating table, and the coordinates of the touch event can correspond to a position of a leg support of the operating table. An updated image can therefore show the operating table having a lifted leg support. In some embodiments, the computer system can process the information obtained about the touch event or initiate extern events, such as a movement of the operating table.

The positional information of a user input are not limited to only coordinates of a touch event on the screen. At least in some embodiments, the positional information can relate to a slide event on the touchscreen. The positional information can be represented by a sequence of coordinates that reflect the slide event on the touchscreen. For example, by an upward slide carried out over a graphic of a leg portion of an operating table, a corresponding upward movement of the leg portion can be initiated. Moreover, an updated image that is generated in response to the detection of such a slide event could show the leg support in a lifted position. Also multi-touch or dual-touch events can be detected and processed in a corresponding way.

In some embodiments, the computer system is configured to determine, based on user input received from the remote control that a state of the medical device is to be changed from a current state to a new state. For example, a touch event or a sliding movement on the touchscreen could be interpreted as a user command to change a setting or a state of the medical device from a current state to a new state. The input event can also be a multi-touch or dual-touch event.

In some embodiments, the computer system is configured to generate updated image data, which includes at least one updated image of the medical device which shows the medical device in the new state, and wherein the computer system is configured to provide the updated image data to the remote control. For example, the medical device can be an operating table and the new state can relate to an uplifted position of a leg support of the operating table. The leg support can have been selected via the remote control to be moved. The updated image data can be provided to the remote control by sending updated data from the computer system to the remote control. The updated data can include the updated image data and, optionally, displaying instructions, such as an anchor point for displaying the at least one updated image.

In some embodiments, updated image data can be buffered on the computer system and/or on the remote control.

In some embodiments, the at least one graphical user interface element includes an input option or a set of input options which is displayed on the screen.

In some embodiments, the computer system is configured to provide image data to the remote control, which includes at least one visualization of the medical device for each input option of the set of input options.

In some embodiments, the computer system provides control means for controlling the graphical user interface. In some embodiments, a graphical user interface logic is provided by the computer system, and the logic can be used by the remote control for displaying the graphical user interface on its screen. A GUI can be divided into the graphics and layout displayed to the user, and the logic which determines how the GUI responds to the user's interaction. In some embodiments, the computer system is configured to control both aspects of the GUI. The location where computer-generated GUIs are displayed may be a predetermined subset of the remote control screen.

In some embodiments, a set of input options is provided to a user and the image data of the data provided to the remote control includes at least one view of the medical device for each input option of the set of input options. For example, in order to address potential issues with reaction time after user input, the computer system can provide at least one view of the medial device for each input option. The views can be provided as images or animations corresponding to a series of sequential images, and the view can be prebuffered on the remote control.

In some embodiments, the image data further includes instructions that cause the remote control, in response to the user selecting an input option, to display the at least one view of the medical device which is related to the selected input option. For example, a prebuffered view can be displayed by the remote control on the screen directly in response to a specific user input, as for example specified by a graphical user interface logic provided by the computer system.

For example, the computer system of an operating table may instruct the remote control to replace an image representation of a GUI button with an image that shows a pressed-down button, when the user presses in a region, which is defined by the computer system, on the touchscreen. In this way, the remote control can appear to respond more quickly to user input, without waiting for the computer system to receive and process the user input. The computer system can send an updated image to be displayed. The remote control can show an immediate response to the user by depicting one or more prebuffered images and until the at least one updated image arrives. The user experience is thereby enhanced. Similarly, the computer system can instruct the remote control, in particular by use of the displaying instructions provided in updated data that is sent to the remote control, to replace certain graphics or show prebuffered images in response to the user pressing a specific hardware button on the remote control.

In some embodiments, different input options are provided in different regions of the touchscreen. The different input options can for example be visualized in an intuitive way, so that the user can easily identify the input options.

In some embodiments, the communication interface is a wireless communication link/interface, which is provided by a WLAN. Therefore, a large bandwidth can be ensured. A Wifi connection can be created between the computer system and the remote control. The computer system can create an "Access Point" that the remote control can connect to when the functionality is needed. Alternatively, the communication interface can be a wired communication link/interface.

In some embodiments, the computer system is a Transmission Control Protocol (TCP) server and the remote control is a TCP client connected to the TCP server via the wireless communication interface. Based on a TCP/IP client-server model, the communication between the remote control and the computer system can be implement in an efficient way. In conjunction with a WLAN connection a real-time or close to real-time user experience can be obtained.

The computer system can be a computer system that is associated with the medical device, and the computer system can be arranged in a housing of the medical device. The computer system can be connected to another computer system, in particular within the medical device, which controls the operation of the medical device.

The graphical user interface, when executed on the remote control, can be configured to control at least one of the following: the screen, a processor, a storage, a database on the storage of the remote control. The processor can be a CPU (for central processing unit) of the remote control. The storage can be a non-volatile storage of the remote control.

At least in some embodiments, the images or other graphical user interface elements provided by the computer system to the remote control are stored in a database on the computer system. Some images can reflect different states of the medical device. For example, when the medical device is an operating table, some images might show the medical device with an upwardly lifted leg support, while other images show the medical device with a lowered leg support. The images can be generated or provided in real time. Furthermore, a 3D-rendering of the medical device or parts thereof can be implemented.

In some embodiments, the remote control is configured similar as a web browser using its screen to display graphic elements and in particular images provided by the computer system, which is configured similar as a web server. The computer system, which functions similar as web server, can instruct the remote control of what type of graphic elements shall be displayed, and where on the screen, and the computer system can send any image or animation, which corresponds to a sequence of images. The remote control can use instructions provided by the computer system and display the image or animation onscreen as instructed.

At least in some embodiments of the present invention, a medical system in accordance with the present invention comprises:
a medical device, in particular an operating table, having a computer system, in particular for controlling operation of the medical device;
a remote control for remotely controlling operation of the medical device, the remote control comprising a screen;
the computer system and the remote control being configured to communicate with each other via a communication interface; the computer system being configured to provide data to the remote control over the communication interface, the data comprising image data of at least one image and optional displaying instructions for displaying the at least one image on the screen; and
the remote control being configured to display, in particular in accordance with the image data and the displaying instructions, the at least one image on the screen.

The computer system, which can control operation of the medical device, in particular via a further computer system of the medical device, can have direct control over the remote control and in particular over the screen of the remote control. The computer system is enabled to provide data to the remote control that includes image data and displaying instructions on the basis of which the remote control displays the at least one image that is associated with the image data on the screen. The computer system can interact with a user and provide visual information to the user in a way that is not necessarily programmed into a GUI software of the remote control. Rather, the remote control is enabled to display the at least one image based on the data that is provided by the computer system.

At least in some aspects, the invention relates to a medical device, in particular for use in a medical system in accordance with the present invention. At least in some embodiments, the medical device comprises a computer system which is configured to provide a graphical user interface to the remote control to control at least a portion of a screen of the remote control by the graphical user interface and to initiate the display of at least one graphical user interface element on the screen of the remote control. The graphical user interface element can be an image.

In some embodiments, the computer system can be configured to provide or generate data which comprises image data of at least one image and displaying instructions for displaying the at least one image on a screen. The computer system may be configured to send the data over a communication interface, in particular to a remote control.

The remote control can be a dedicated remote control. The primary purpose of the remote control is therefore to control the medical device. At least in some embodiments, it is not intended that the remote control is used for purposes that are unrelated to the medical device to which it is dedicated. However, in some embodiments, the remote control can be employed, in particular by use of the computer system, to control other devices as well.

The computer system of the medical device can be connected with another, second computer system and obtain the at least one graphical user interface element, for example relation to the mentioned data or the updated data, from the second computer system. In this regard, the computer system can act as an intermediate station in between the remote control and the second computer, which can control the remote control, in particular via its graphical user interface that is used to control the remote control. In some embodiments, the (first) computer system is part of an operating table, and the second computer system is part of a different second medical device (such as an operating light), and the operating table computer system acts as an intermediate between the remote control and the second medical device.

At least in some aspects, the invention also relates to a remote control, in particular for use in a system in accordance with the present invention. In at least some embodiments, the remote control comprises a screen, such as a touch screen, and the remote control is configured to receive data, in particular from a computer system over a wireless communication interface, which comprises image data of at least one graphical user interface element, in particular an image, and displaying instructions for displaying the at least one graphical user interface element on a screen. The remote control can be configured to display, in accordance with the image data and the displaying instructions, the at least one graphical user interface element on the screen.

At least in some embodiments, the remote control is configured to execute a graphical user interface provided by the computer system that controls at least a portion of the screen of the remote control.

At least in some aspects, the invention also relates to a method of operating a medical system, which comprises a medical device, in particular an operating table, with a computer system and a remote control, the method comprising:
controlling at least a portion of a screen of the remote control by a graphical user interface of the computer system, and
initiating the display of at least one graphical user interface element on the screen of the remote control, wherein the graphical user interface of the computer system is executed on the remote control.

At least in some aspects, the invention also relates to a method of operating a medical system comprising a medical device, in particular an operating table, with a computer system and a remote control. At least in some embodiments, the method comprises:
generating, by use of the computer system, data which comprises image data of at least one image, in particular of the medical device, and displaying instructions for displaying the at least one image on a screen; and
sending, by use of the computer system, the data to the remote control over a wireless communication interface, wherein the remote control and the computer system are connected with each other via the wireless communication interface.

The method can further include the step of displaying, for example based on the image data and the displaying instructions, the at least one image on the screen of the remote control.

The invention also relates to a medical system comprising an operating table with a computer system for controlling operation of the operating table. The computer system of the operating table is configured to be connectable or is connected with a plurality of electronic devices via a wireless or a wire-based connection between each electronic device and the computer system. The computer system can further be configured to send control instructions to the remote control.

At least in some embodiments, the communication between the computer system and each electronic device is based on the IEEE 11073 service-oriented device connectivity (SDC) family of standards, which define a communication protocol for point-of-care medical devices. A manufacture-independent communication can thereby be achieved between the computer system of the operating table and other medical or non-medical electronic devices connected to the computer system.

Although some of the present claims are related to an operating table, the invention can also be related to other medical devices. At least in some embodiments, the invention can be related to a medical system with a medical device, such as operational lights, and a remote control, and a method of operating a medical system with a medical device and a remote control.

Various embodiments in accordance with the present disclosure will be described with reference to the drawings, in which:
- Fig. 1: shows a block diagram of a medical system in accordance with at least some aspects of the present invention.
- Figs. 2a-f: illustrate an example communication process in a medical system in accordance with at least some aspects of the present invention.
- Figs. 3a-f: illustrate another example communication process in a medical system in accordance with at least some aspects of the present invention.
- Figs. 4a-i: illustrate another example communication process in a medical system in accordance with at least some aspects of the present invention.
- Fig. 5: shows a block diagram of a medical system in accordance with at least some aspects of the present invention.
- Fig. 6: shows schematically an operating table and a remote control for the operating table.

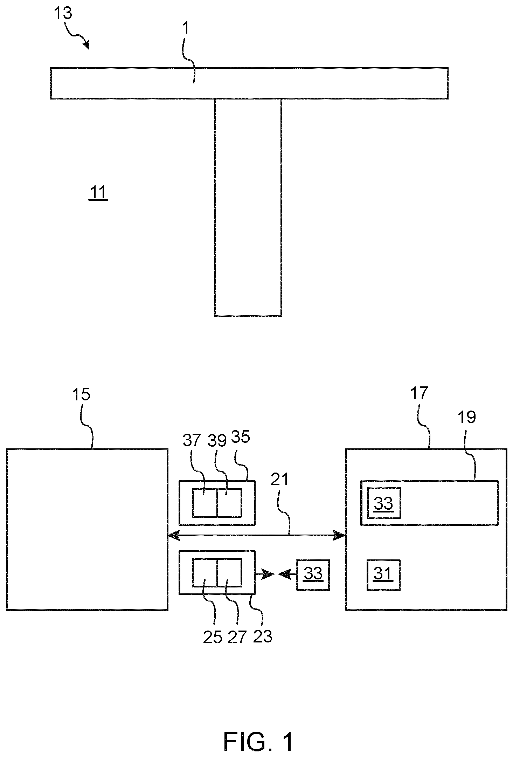

The medical system 11 of Fig. 1 comprises a medical device 13, which is in the shown example an operating table. The medical device 13 includes a computer system 15, which can be connected to a further computer system (not shown) used for controlling operation of the medical device 13. In systems where the medical device 13 is an operating table, the computer system 15 might be in or on the base or the column of the table. The system 11 includes a remote control 17 for remotely controlling operation of the medical device 13. The remote control 17 comprises a screen 19, which can be a touchscreen. Moreover, the computer system 15 and the remote control 17 are configured to communicate with each other via a wireless communication interface 21. Alternatively, the interface can be a wired communication interface, but it is assumed in the following that the interface is a wireless communication interface.

The computer system 15 are configured to provide via the communication interface 21 a graphical user interface to the remote control 17, and the remote control 17 is configured to execute the graphical user interface. The computer system 15 is further configured to control at least a portion of the screen 19 by use of the graphical user interface, when executed in the remote control 17, and to initiate the display of at least one graphical user interface element on the portion of the screen of the remote control 17.

In some embodiments, the computer system 15 is configured to provide the at least one graphical user element to the remote control 17 by use of data 23 which is sent to the remote control 17 over the wireless communication interface 21. The data 23 comprises image data 25 of at least one image, in particular an image of the medical device, and optional displaying instructions 27 for displaying the at least one image on the screen 19 of the remote control 17.

A location where the image is displayed can be predetermined. Then, no displaying instructions 27 would be required. Thus, in some cases, the remote control might only need the image(s) to be shown or streamed. It does not need displaying information, like an "anchor point". For instance, the remote control can in some embodiments have a defined box onscreen where it would put an image when it is received. Also, if the computer system controls the entire screen, there is no need to provide displaying instructions, because the image can be fit to the entire screen.

The remote control 17 is configured, in particular by use of the graphical user interface, to display the at least one image on the screen 17 in accordance with the image data 25 and the displaying instructions 27. Thus, in some embodiments, the at least one graphical user interface element corresponds to at least one image that is provided by the computer system 15 to the remote control 17 and displayed on its screen 19.

At least in some embodiments, the remote control 17 is a dedicated remote control whose primary purpose is to enable remote control means for the medical device 13. The remote control 17 is configured, in particular by use of the graphical user interface provided by the computer system 15, to accept user input 33 from a user either via the screen 17, when it is a touchscreen, or via physical buttons 31, and to communicate the user input 33 to the computer system 15, which interprets the user input 33 and which can initiate the steering of the medical device 13 accordingly.

At least in some embodiments, the wireless communication interface 21 is provided by a WLAN (for wireless local area network). The computer system 15 can create an access point to the WLAN that the remote control 17 can connect to in order to establish the communication interface 21. At least in some embodiments, the computer system 15 is configured as a TCP server and the remote control 17 is configured as a TCP client, which is connected to the TCP server via the wireless communication interface 21.

At least in some embodiments, the computer system 15 can receive user input 33, received at the touchscreen 19 or at a physical button 31 of the remote control 17. For example, the user input 33 can trigger a change of a setting of the medical device 13. For example, a leg support of an operating table can be moved to an upwardly lifted position in response to receiving the user input. The computer system 15 can generate updated image data 35 in accordance with the received user input 33. In accordance with the mentioned example, the updated image data 35 can include at least one updated image 37 of the operating table in which the leg support is shown in the upwardly lifted position. The updated image data 35 can be provided to the remote control 17 and the at least one image 37 can be shown on its screen 19, in accordance with updated displaying instructions 39 included in the updated image data 35. Thus, a graphical user interface element can relate to the updated image 37.

At least in some embodiments, the screen 19 is a touch screen and the computer system 15 is configured to receive positional information of a user input 33 on the touch screen 19. The positional information can include coordinates of the user input 33 as occurred on the touchscreen 19. The positional information can further include a pressure value related to the touch event. The computer system 15 can interpret the positional information and initiate a movement of a part of the medical device. Furthermore, updated image data 35 can be generated and provided to the remote control 17 for display. The updated image data 35 can include at least one image of the medical device or at least the part of the medical device which reflects that the movement has been carried out. A graphical user interface element can therefore relate to the updated image.

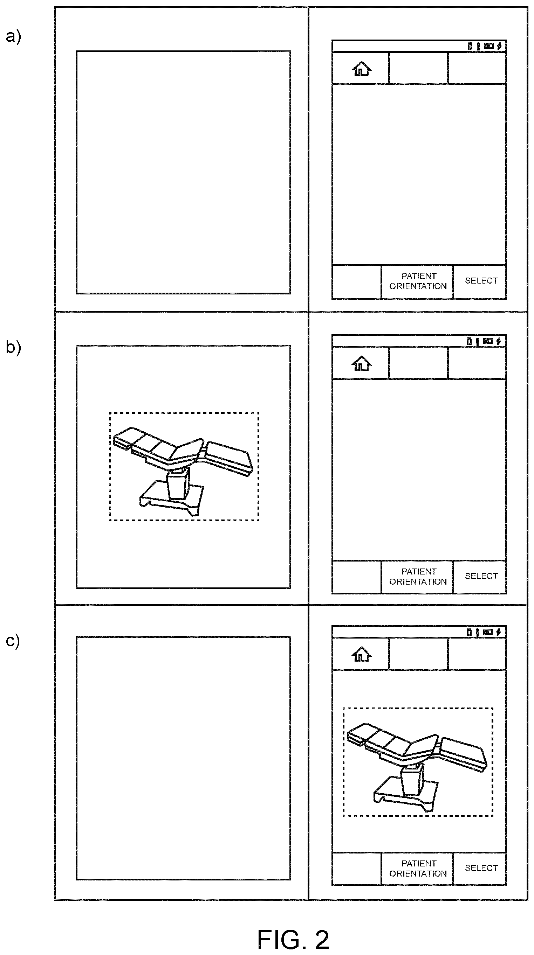

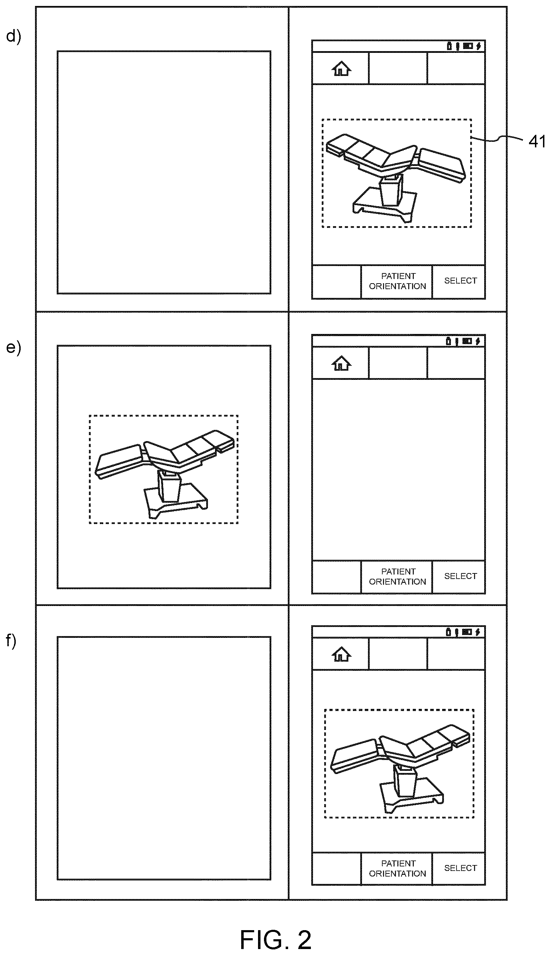

Figs. 2 a-f relate to an example medical system in which the medical device is an operating table and the figures illustrate an example communication process. In each of Figs. 2 a-f, the left part of the figure shows, if applicable, a graphical user interface element, in particular an image, provided by the computer system of the operating table, and the right part of the figure shows a view of the screen of the remote control.

As shown in Fig. 2a, the computer system has not yet determined an image of the operating table, so the left part of Fig. 2a is blank. As can be seen on the right part of Fig. 2a, the remote control displays a basic screen to the user, without a table preview. The basic screen can be provided by the remote control without interaction with the computer system. Fig. 2a describes, for example, an instance during an initial use of the operating table and the remote control.

As illustrated on the left part of Fig. 2b, the computer system generates an image, for example a preview, of the operating table in its current state. The image can for example be a jpg image. Image data including the image and optional displaying instructions is sent to the remote control. The optional displaying instructions can include or consist of anchor coordinates that indicate where the image is to be shown on the screen. In some embodiments, the location for displaying the image is predetermined, so that no displaying instructions are required. The right part of Fig. 2b indicates that the remote control still shows its basic screen.

The right part of Fig. 2c shows that the remote control has received the image data and displays the image to the user on its screen. The left part of Fig. 2c indicates that the computer at this instance generates no new image data. In this example, the computer system has only provided an image (of the table) for part of the remote screen. In the example, the remote has provided buttons and other images in other areas of the screen.

The left part of Fig. 2d indicates that the computer still generates no new image data. The right part of Fig. 2d indicates that the user touches the screen. The touch event is visualized by use of a dotted rectangle 41. The remote control sends data about the touch event to the computer system. The data about the touch event can include position and/or movement information about the touch event, which can be provided in form of coordinates of the touch event on the screen. In some embodiments, the coordinates of the touch event can correspond to the coordinates of the geometrical center of the rectangle 41.

As shown in the left part of Fig. 2e, after reception of the data about the touch event, the computer system generates updated image data including a new image of the operating table and optional displaying instructions, for example including an anchor point for the display of the new image on the screen of the remote control. The touch event can trigger a state change of the operating table from its current state to a new state. For example, a leg support is in an upwardly lifted position in the new state. In another example, as indicated in Fig. 2e, the patient orientation can be flipped in the new state. As shown in the right part of Fig. 2e, the remote control has not yet received the updated image data and its display shows the basis screen after the detection of the touch event. Alternatively, the remote control screen might display the old image, for example as shown on the right of Fig. 2d, until a new image is received.

As shown in the right part of Fig. 2f, the remote control received the updated image data and shows the new image, in accordance with the anchor point, on the display. The left part of Fig. 2f is blank, as no new image is generated by the computer system.

Thus, as illustrated with regard to Figs. 2a-f, as the operating table moves, the remote control shows an updated image of the table to reflect the newest position. For instance, the distance between the table top and the base of the table may change on the image. Similarly, the angle of the legs plates or head plate might be updated, etc. As the images are provided by the computer system, an improved approximation or a real 3D visualization of the table can be provided, even though the remote control does not have to provide more processing power. The computer system in the operating table has enough processing power to do a real or improved 3D rendering of the table. It also has much more storage capacity, which will allow it to have a 3D rendering of all of the accessories. Finally, its connection to the internet will allow constant updating to include new accessories that enter the market after the table is released. In some embodiments, 2D images of the table can be provided instead of 3D images.

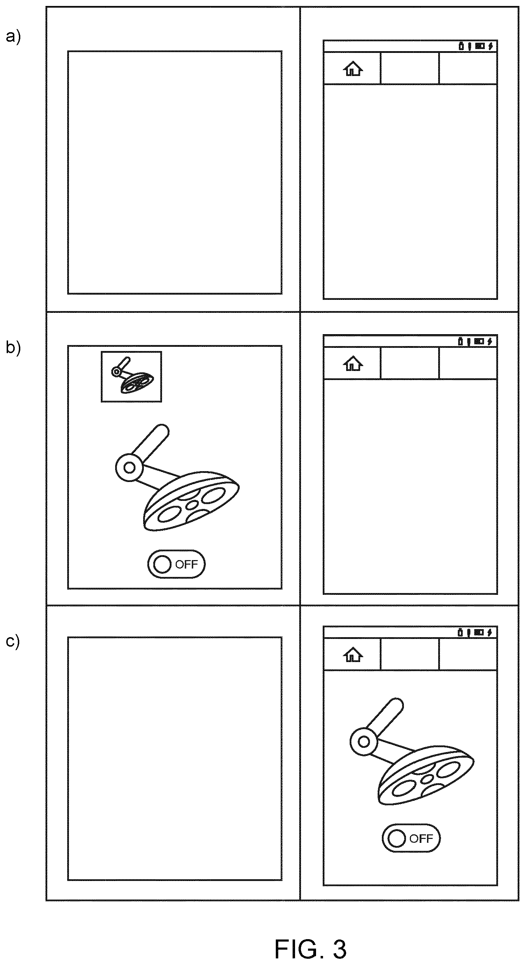

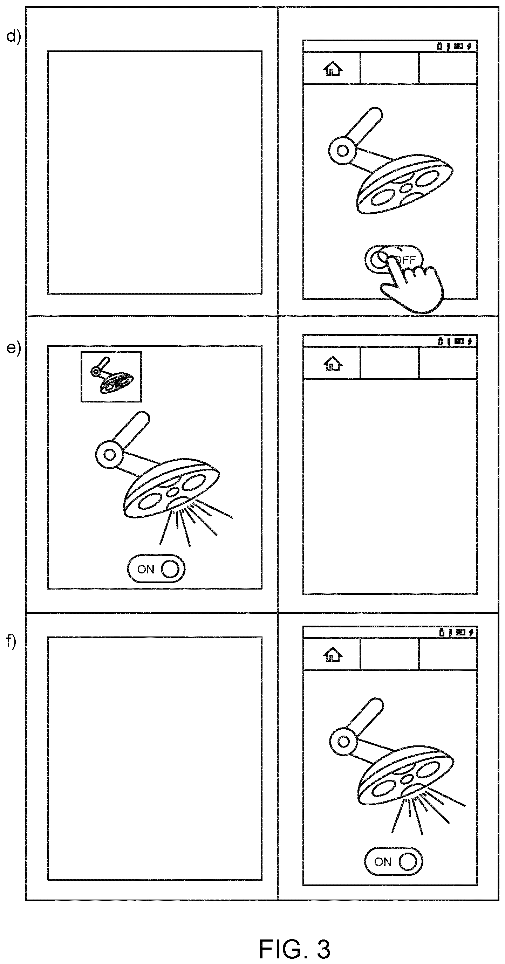

In another example, illustrated with reference to Figs. 3a-f, the second medical device is an operating light which is controlled by the computer system of the operating table (first medical device) and its remote control. The left part in each of the Figs. 3 a-f serves to illustrate an action that is carried out by the computer system, while the right part shows a view of the display of the remote control. The left part of Fig. 3a is blank, indicating that no action with regard to the control of the operating light is taking place at the computer system, and the remote control displays the basic screen as shown in the right part of Fig. 3a.

Fig. 3b illustrates on the left part that the computer system (in the operating table/first medical device) generates image data with a graphical user interface element in form of an image, such as a jpg image, including a graphic of the operating light, a graphic of an "OFF"-icon and displaying instructions that include or consist of anchor coordinates. The "OFF"-icon indicates that the operating light is switched off. The graphics are placed on a transparent background mask. The image data is sent by the computer system to the remote control, which still shows the basic screen as shown in the right part of Fig. 3b.

As shown in the right part of Fig. 3c, the remote control receives the image data and displays, in accordance with the anchor coordinates, the graphic of the operating light and the graphic of the "OFF"-icon on the transparent background mask on the screen. This can in particular be done by use of a graphical user interface provided by the computer system and that controls at least a portion of the screen of the remote control. The left part of Fig. 3c indicates that the computer system does not generate any new image data at this instance.

According to the left part of Fig. 3d, the computer system still does not generate any new image data. As further illustrated in the right part of Fig. 3d, the user touches the screen on the location of the "OFF"-icon. The remote control can detect positional information of the touch event, for example in form of coordinates of the touch event on the screen. The positional information is sent from the remote control to the computer system in the operating table/first medical device.

As shown in the left part of Fig. 3e, the computer system interprets the positional information and determines, based on the received positional information that the light is to be switched on. The computer system generates an updated image which shows a graphic of a glowing operating light and a graphic of an "ON"- icon on a transparent background mask. Updated image data which comprises the updated image and displaying instructions, such as an anchor point, are sent to the remote control. At this instance, the remote control shows the basic screen as illustrated in the right part of Fig. 3e. Alternatively, it can show the old image as shown on the right of Fig. 3d.

According to the right part of Fig. 3f, the remote control has received the updated image data and displays the updated image in accordance with the associated anchor point. As mentioned before, the anchor point can optionally be provided. The position on which the updated image is shown could also be predetermined. At this instance, the computer system does not generate new image data as illustrated by the blank part on the left part of Fig. 3f.

The home button, which is shown in the left corner of the right part of each of Figs. 2a-f and 3a-f, can be a button which enables a user to switch back to the remote control's own graphical user interface, whereas otherwise the display is controlled by the computer system as described above. At least in some embodiments, the display of the remote control can be controlled by a graphical user interface of the computer system, and hence, the remote control, or at least its display, can be regarded as an extension of the computer system. The remote control thereby can serve to display image data provided by the computer system, and to provide user input directly to the computer system, in particular without interpreting the user input.

As illustrated by way of example, the computer system of the table can be in direct communication with other devices in the operating room (for example one or more operating lights). The remote control will not have direct communication with those devices. The table can also be able to use its internet connection, and to get software updates, such as the latest control profiles for newly installed equipment in the operating room. The remote control is not required to have this capability.

As the computer system can control the remote control, the remote control does not need to be updated when new control profiles are installed on the computer system of the operating table. Moreover, the remote control does not necessarily need to be aware of context or events that are happening when it is being used to control another device in the operating room. It can simply show the images the table computer tells it to, and the return the user input events to the computer system of the table, which will be able to handle the rest.

This can be important when a remote control needs to be replaced in the middle of an operation (because it malfunctions, or is dropped, etc.). If the replacement remote (taken from storage) has not been updated to the latest software, then it might not have all the device profile information needed to control the other devices in the room. However, in accordance with embodiments of the present invention, this will not be a problem, because the remote does not need to have this information.

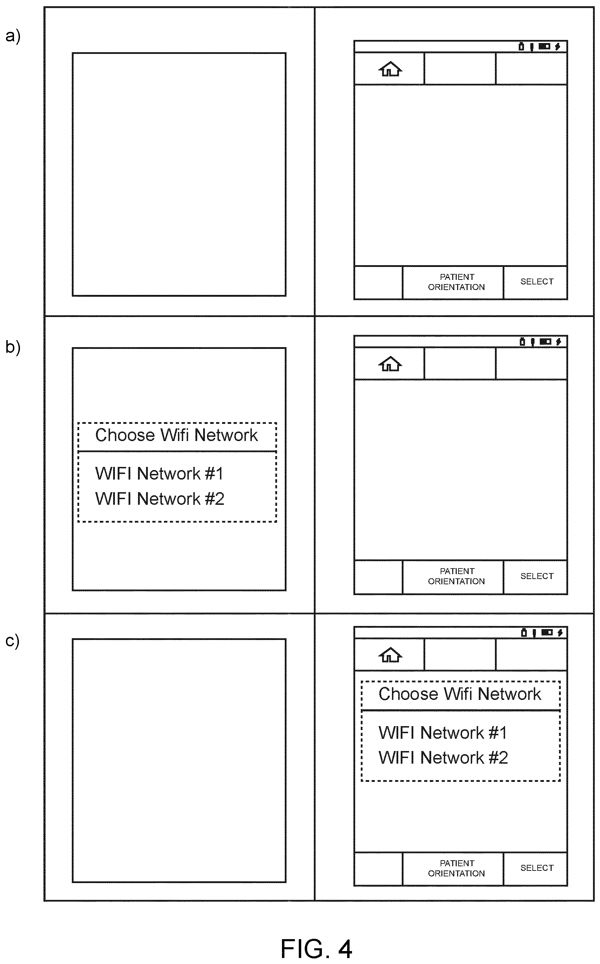

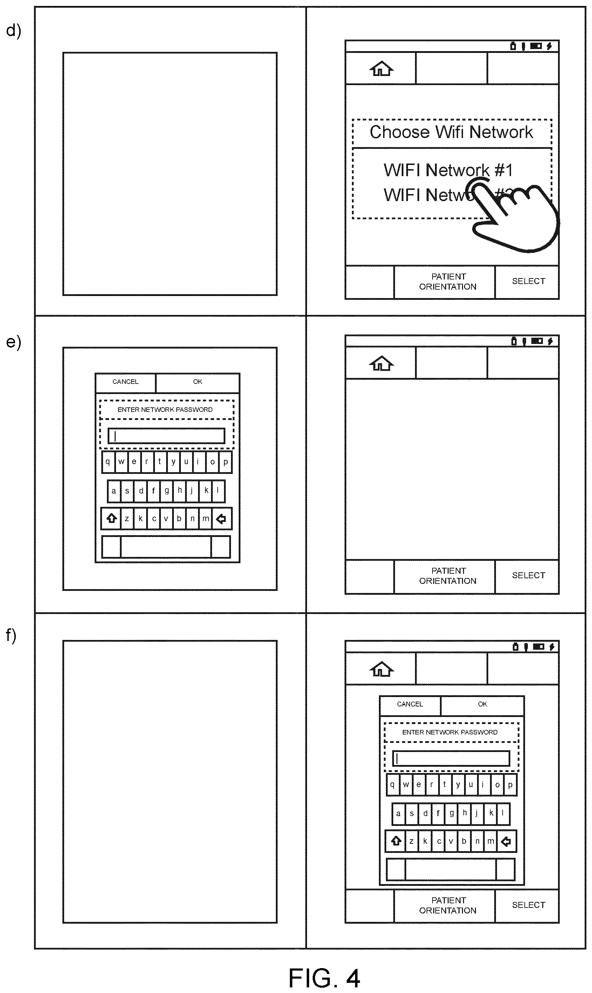

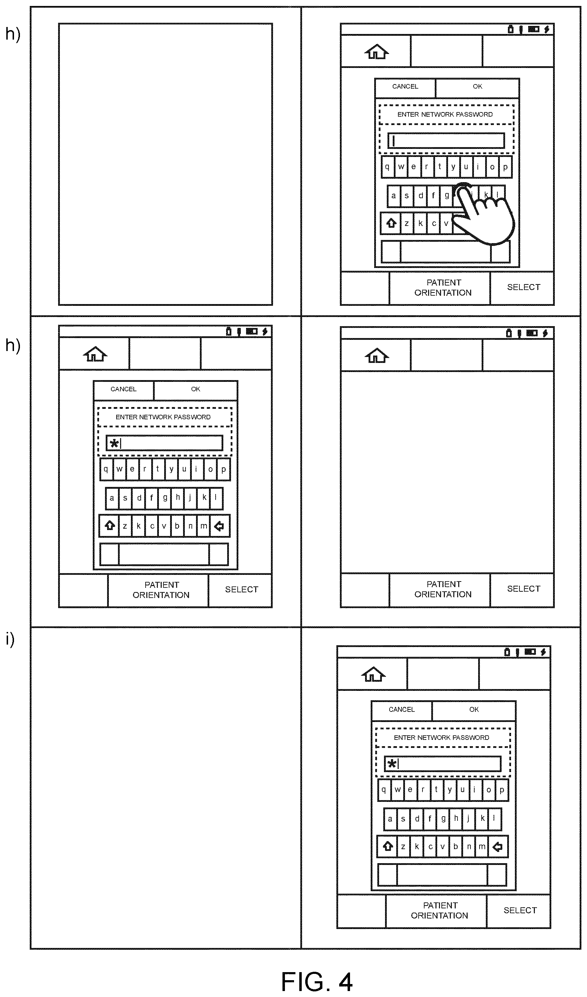

Figs. 4 a-i relate to another example medical system in which the medical device is an operating table and the figures illustrate an example communication process. In each of Figs. 4 a-i, the left part of the figure shows, if applicable, a graphical user interface element, in particular an image, provided by the computer system of the operating table, and the right part of the figure shows a view of the screen of the remote control, of which at least a portion is controlled by the computer system via its graphical user interface.

As shown in Fig. 4a, the computer system has not yet determined a GUI to display, so the left part of Fig. 4a is blank. As shown in the right part of Fig. 4a, the remote control displays a basic screen to the user. The basic screen can be provided by the remote control without interaction with the computer system. Thus, in some embodiments, the remote will simultaneously display elements provided by the remote control and elements provided by the computer system in a table in at least some circumstances.

As illustrated on the left part of Fig. 4b, the computer system generates a graphical user interface element, which in this example comprises a text field by which the user is asked to choose between "Wifi Network #1" and "Wifi Network #2". The two mentioned networks can be "real" networks to which the computer system can connect. The right part of Fig. 4b indicates that the remote control still shows its basic screen.

The right part of Fig. 4c shows that the remote control has received the graphical user interface element and displays it to the user on the screen. The left part of Fig. 4c indicates that the computer at this instance provides no new graphical user interface element.

The left part of Fig. 4d indicates that the computer still generates no new graphical user interface element. The right part of Fig. 4d indicates that the user touches the screen at the position above "Wifi Network #2". The remote control sends coordinates or the object of the touch event to the computer system. In some embodiments, the coordinates of the touch event can correspond to the coordinates of the geometrical center of the touch event. The remote does not need to interpret the meaning of the touch event or generate a comment in response to the touch event.

Thus in some embodiments the computer (which can be in a table) generates a list of options (which may relate to the table or another operating room device) and instructions for graphically presenting the list of options to a user. The computer can send instructions for graphically presenting the list of options to the remote, which displays the list of options on at least part of the remote display. The remote may then record a touch event in relation to the list of options, and relay the touch event back to the computer/table (without interpreting the touch event). The computer then interprets the touch event in relation to the list of options (which may be a selection of one of the options), and generates and transmits instructions in response to the touch event and/or in response to the selection from the list of options. Lists could be provided as a set of buttons, as a simple list of options, or as a drop-down menu, for example. In some embodiments, the list of options may be yes/no for a question, or on/off for a feature or a device.

Returning to the specific Figure 4 example, as illustrated in the left part of Fig. 4e, the computer system has received information about the touch event, in particular the coordinates, and the computer system interprets the event. The computer system generates an updated graphical user interface element, through which the user can enter a password for the selected network "Wifi Network #2". Data that includes the graphical user interface element and an anchor point for displaying the graphical user interface element on the screen is sent to the remote control. At this process step, the screen of the remote control does not yet show the updated graphical user interface element, as shown on the right of Fig. 4e.

The left part of Fig. 4f indicates that the computer generates no new graphical user interface element. The right part of Fig. 4f indicates that the remote control has now received the updated graphical user interface element of Fig. 4e and displays it on the screen as instructed via the anchor point. I this example, the graphical user interface element includes a virtual keyboard including a plurality of letter keys. Number keys are also contemplated.

As shown on the right of Fig. 4g, the user can input the password at the remote control. As the user types on the screen with an onscreen display, the remote control sends the associated touch events to the computer system. The right portion of Fig. 4g indicates that the remote does not in this example generate a new updated graphical user interface element in response to the user typing/touch events.

Fig. 4h indicates on the right part that the computer system process each touch event, converts it to a keyboard press internally, and prepares an updated graphical user interface element that reflects each touch event by a "*" that is added to the input line. The right part of Fig. 4h indicates that a touch event is not yet reflected on the screen of the remote control, while the right part of Fig. 4i indicates that the user sees an updated graphical user element, which indicates that the first password character has been typed in. The left part of Fig. 4i is left blank. In alternative embodiments, the remote might supply simple graphics to acknowledge user inputs (without necessarily interpreting their meaning).

Further with regard to Figs. 4a-i, a computer system of an operating table can nowadays have more software and therefore more configuration than ever before. Some of this configuration can be done over the remote control. For example, as illustrated, a Wifi network password can be provided via the remote control. Moreover, some configuration screens can be complex. These screens may need to be updated over time, for example as a manufactured of such operating tables learns more about the users' needs. For instance, in the future, users might be able to select, configure, and connect the computer system of the operating table to the hospital Wifi network using the remote control. To enable this, the computer system first needs to scan the Wifi environment and present a list of available networks to the user. The user will then select the network, and type in the password over the remote. Initially, this functionality might be basic, providing only the list, and the ability to type a password using a minimal keyboard on the remote. However, for instance, additional keyboards with special characters might be included, or additional capabilities such as setting up a Proxy server or VPN might be included.

Thus in some embodiments the computer (which can be in a table) generates a graphic for requesting and receiving alphanumerical input from a user. The computer can send instructions for graphically requesting and receiving the alphanumerical input to the remote, which displays a graphic for requesting and receiving alphanumerical input. In this example, the graphic is in the form of a virtual touchscreen keyboard. The remote may then record one or more touch events with respect to the graphic, and relay the one or more touch events back to the computer/table. The computer then interprets the one or more touch events as alphanumerical user input, and generates and transmits instructions and/or information in response to the alphanumerical user input. In this example the alphanumerical input is a network password, but other types of alphanumerical input are also contemplated. The remote does not necessarily need to "understand" the alphanumerical input at all or translate it onto commands.

As described with reference to Figs. 2a-f, 3a-f, and 4a-I the remote control can display the images or more generally the graphical user interface elements and return user input without having any awareness of the actual interaction or commands between the user and the table. It displays the images as instructed by the table, for example based on VNC.

In some embodiments, the computer system can send the remote prebuffered images (or other graphical user interface elements) or a sequence of prebuffered images (or graphical user interface elements), which corresponds to an animation, and associated instructions to display them directly on the screen of the remote control in response to a specific user input. For example, the table may instruct the remote control to replace an image representation of a GUI button with a pressed version of the image, when the user presses in a defined region of the screen. In this way, the remote control can appear to more quickly respond to user input, without waiting for the computer system to receive and process the touch event, and then send the next image to be displayed. Similarly, the computer system can instruct the remote control to replace certain graphics in response to the user pressing a specific hardware/physical button.

In some embodiments, the computer system only provides images for part of the remote control screen, not the entire screen. For example, in some embodiments, the remote control is configured to locally generate one or more graphic elements, in particular GUI elements, on a first portion of the screen at the same time as the computer system controls and displays GUI elements on the second portion of screen. In some embodiments, the remote control can place graphic elements outside a displayed image provided by the computer system. In some embodiments, the remote control layers graphic elements in front of or behind a displayed image provided by the computer system. In some embodiments, the remote control can provide a button, such as a home button, which takes the user back to the home screen of the remote control. Such button can be placed at the top left corner of the screen. In some embodiments, the computer system can specify a transparent area within an image to be displayed. When the image is shown on the screen of the remote control, the transparent area can be used to visualize a graphic element, which is placed by the remote control at a layer behind the transparent area.

In some embodiments, a single universal remote control is usable with several different types of operating table. Each table model can be unique and can have special needs and capabilities. The remote control can be used with tables in many specialized setups. For instance, it can be used in a so-called "Hybrid Operating Room" where the operating table and imaging systems from different manufacturers work together. As the display of the remote control can be controlled via the computer system as described before, situation specific interfaces need only be programmed into the computer system of an operating table (and not necessarily in the remote), which then communicates with and controls the remote control. In this way, there is no need to program every possibility into the remote control. Thus in some embodiments, a single remote control is usable with a plurality of different types of medical tables. The respective tables provide, and the remote receives and displays, graphics and functional options which are appropriate for the type of table which is being controlled.

At least in some embodiments, the remote control is sold with the medical device, such as the operating table, and its primary function is to control the medical device. However, at least in some embodiments, the remote control can be linked, in particular via the computer system and the functionality as described before, to other devices, such as operational lights. In some embodiments, the communication can be direct between the lights and the remote control, without being facilitated by the computer system of the medical device as an intermediary.

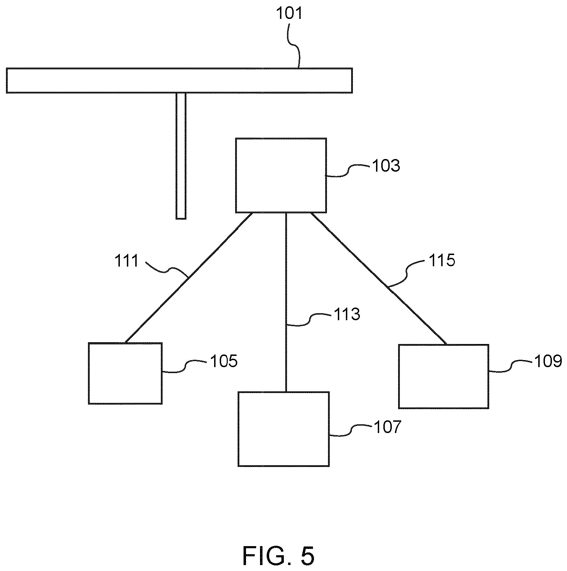

The medical system schematically shown in Fig. 5 comprises an operating table 101 having a computer system 103 for controlling operation of the operating table 101. The computer could be located in or on the table base, in or on the table column, or another location on or inside of the table. The computer system 103 is configured to be connected or is connected with a plurality of electronic devices, here three electronic devices 105, 107, and 109, which can be medical or non-medical devices. For example, other devices 105, 107, 109 in an operating room with the table. A wireless or a wire-based connection 111, 113, 115 can be or is established between each electronic device 105-109 and the computer system 103. The computer system 103 of the operating table 101 can therefore act as a central hub and the electronic devices 105-109 can be regarded as hosts of a star network formed by the computer system 103 and the electronic devices 105-109. A remote control can be used to control the electronic devices 105-109 via the computer system 103.

The communication between the computer system 103 and each electronic device 105-109 can be based on the IEEE 11073 service-oriented device connectivity (SDC) family of standards.

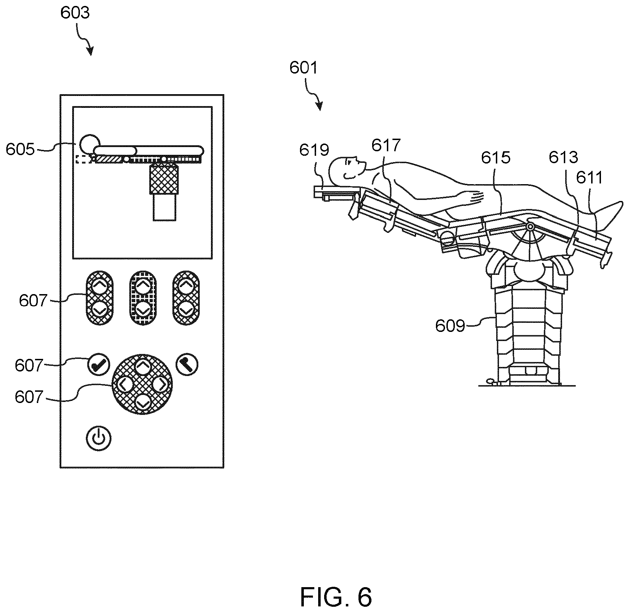

Fig. 6 shows schematically an example of an operating table 601 and a remote control 603 for the operating table. The remote control 603 includes a screen 605, which can be a touchscreen, and several buttons 607. The operating table 601 includes a table column 609, which can be adjusted via one or more actuators in the vertical and horizontal directions. A patient support surface 611 rests on the table column 609, which includes a lower leg segment 613, an upper leg segment 615, a back segment 617, and a head segment 619. The segments are connected to one another by articulation, such that the patient support surface 611, on which the patient lies, can be brought into a desired shape. The lower leg segment 613, the upper leg segment 615, and the back segment 617 can be adjusted through one or more actuators. The head segment 619, on the other hand, can be adjusted by hand in this configuration. In some embodiments, the head segment 619 can also be adjusted through one or more actuators. The buttons 607 of the remote control 603 are assigned to different table components. A more detailed example of an operating table and a remote control is described in WO 2016/131659 A1.

The examples disclosed in this application are non-limiting. It will be appreciated that the general concepts and specific examples disclosed here may be used in various combinations.

Exemplary embodiments and variants in accordance with the present disclosure are described in the following list of items and options:
Item 1. A medical system comprising:
   a medical device (13), in particular an operating table, having a computer system (15) for controlling operation of the medical device (13);
   a remote control (17) for remotely controlling operation of the medical device (13), the remote control (17) comprising a screen (19);
   the computer system (15) and the remote control (17) being configured to communicate with each other via a wireless communication interface (21);
   the computer system (15) being configured to provide data (23) to the remote control (17) over the wireless communication interface (21), the data (23) comprising image data (25) of at least one image and displaying instructions (27) for displaying the at least one image on the screen (19); and
   the remote control (17) being configured to display, in accordance with the image data (25) and the displaying instructions (27), the at least one image on the screen (19).
Item 2. The medical system of item 1,
   wherein the computer system (25) is configured to receive user input (33) from the remote control (17) and to generate updated image data (35) in accordance with the received user input (33).
Item 3. The medical system of item 1 or 2,
   wherein the screen (19) is a touch screen and the computer system (15) is configured to receive positional information of a user input (33) on the touch screen and to generate updated image data (35) based on the received positional information of the user input (33).
Item 4. The medical system of any one of the preceding items,
   wherein the computer system (15) is configured to determine, based on user input (33) received from the remote control (17), that a state of the medical device (13) is to be changed from a current state to a new state.
Item 5. The medical system of item 4,
   wherein the computer system (15) is configured to generate updated image data (35) which includes at least one view of the medical device (13) which shows the medical device (13) in the new state, and wherein the computer system (15) is configured to provide the updated image data (35) to the remote control (17).
Item 6. The medical system of any one of items 1 to 5,
   wherein the medical device (13) is an operating table (13), the operating table having a plurality of support sections for supporting various parts of a patient, the support sections being movable with respect to each other into different arrangements to support different patient positions of the operating table;
   wherein the operating table (13) is configured to move from a first arrangement to a second arrangement by moving one or more of the support sections in response to receiving a movement signal (33) from the remote control (13);
   wherein the computer system (15) is configured to create an updated image (25) of the operating table in the second arrangement in response to at least one of said movement signal (33) and/or the movement of the operating table from the first arrangement to the second arrangement, and to provide the updated image (25) of the operating table in the second arrangement to the remote control (13).
Item 7. The medical system of any one of the preceding items,
   wherein a set of input options is provided to a user and the image data of the data provided to the remote control (17) includes at least one view of the medical device for each input option of the set of input options.
Item 8. The medical system of item7,
   wherein the image data further includes instructions that cause the remote control (17), in response to the user selecting an input option, to display the at least one view of the medical device which is related to the selected input option.
Item 9. The medical system of item 7 or 8,
   wherein different input options are provided in different regions of the screen.
Item 10. The medical system of any one of the preceding items,
   wherein the wireless communication interface (21) is provided by a WLAN.
Item 11. The medical system of any one of the preceding items,
   wherein the computer system (15) is a TCP server and the remote control is a TCP client connected to the TCP server via the wireless communication interface.
Item 12. A medical device, in particular for use in a medical system in accordance with the preceding items,
   the medical device (13) comprising a computer system (15) which is configured to generate data (23) which comprises image data (25) of at least one image and displaying instructions (27) for displaying the at least one image on a screen (19);
   the computer system (15) being configured to send the data (23) over a wireless communication interface (21), in particular to a remote control (17).
Item 13. A remote control, in particular for use in a medical system in accordance with any one of the items 1 to 10,
   the remote control (17) comprising a screen (19), such as a touch screen, and the remote control (17) being configured to receive data (23), in particular from a computer system (15) over a wireless communication interface (21), which comprises image data (25) of at least one image and displaying instructions (27) for displaying the at least one image on a screen; and
   the remote control (17) being configured to display, in accordance with the image data (25) and the displaying instructions (27), the at least one image on the screen.
Item 14. A method of operating a medical system (11) comprising a medical device (13), in particular an operating table, with a computer system (15) and a remote control (17), the method comprising:
   generating, by use of the computer system (15), data (23) which comprises image data (25) of at least one image, in particular of the medical device (13), and displaying instructions (27) for displaying the at least one image on a screen (19); and
   sending, by use of the computer system (15), the data (23) to the remote control (17) over a wireless communication interface (21), wherein the remote control (17) and the computer system (15) are connected with each other via the wireless communication interface (21).
Item 15. A medical system comprising:
   an operating table (101) with a computer system (103) for controlling operation of the operating table (101), and
   the computer system (101) of the operating table (101) being configured to be connected or being connected with a plurality of electronic devices (105, 107, 109) via a wireless or a wire-based connection (111, 113, 115) between each electronic device (105, 107, 109) and the computer system (103).
Item 16. A system or method:
   wherein the computer system (15) of the operating table (13) is configured to generate a list of options, in particular a list of options relating to operation of the medical system, to generate instructions for graphically presenting the list of options to the user, and to communicate the instructions to the remote control (17);
   wherein the remote control (17) us configured to execute the instructions received from the computer system (15), to display the list of options on the screen (19) of the remote control;
   wherein the remote control is further configured to receive a user input associated with the list of options, the user input comprising a touch event associated with the screen (19), and then to transmit the touch event back to the computer system (15);
   wherein the computer system (15) is configured to receive and interpret the touch event as a selection from the list of options, and to act in response to the selection.
Item 17. A system or method:
   wherein the computer system (15) of the operating table (13) is configured to generate a instructions for a graphic to request and receive alphanumerical input from a user, and to communicate the instructions to the remote control (17);
   wherein the remote control (17) is configured to receive and execute the instructions received from the computer system (15), and to display the graphic for requesting and receiving alphanumerical input on the screen (19) of the remote control;
   wherein the remote control is further configured to receive alphanumerical input from a user, the alphanumerical input comprising at least one touch event associated with the screen (19), and then to transmit the at least touch event back to the computer system (15);
   wherein the computer system (15) is configured to receive and interpret the touch event as alphanumerical input, and to act in response to the alphanumerical input.
Item 18. A medical system or method:
   comprising a remote control (17) for remotely controlling operation of the medical device (13), the remote control (17) comprising a screen (19);
   the computer system (15) and the remote control (17) being configured to communicate with each other via a communication interface (21);
   the computer system (15) being configured to provide data (23) to the remote control (17) over the wireless communication interface (21), the data (23) comprising image data (25) of at least one image and displaying instructions (27) for displaying the at least one image on the screen (19); and
   the remote control (17) being configured to display, in accordance with the image data (25) and the displaying instructions (27), the at least one image on a first portion of the screen (19), wherein a different second portion of the screen (19) is not provided or controlled by the image data (25). In some embodiments, the second portion of the screen in controlled by the remote control (17) independent of the image data (25) received from the computer system (25).
Item 19: A medical system or method according to one of items 1-18, wherein the computer system (15) is configured to control operation of the table (13) or
   wherein the computer system (15) is connected to a further computer system of the table which is configured to control the operation of the table (13).
Item 20: The medical system of any one of the preceding claims,
   wherein the graphical user interface provided by the computer system (15) is displayed only on a first part of the screen (19), and images on a different separate part of the screen (19) are provided by the remote control (17).

The terms "communication link" (21) and "communication interface" (21) are considered equivalent and interchangeable herein. Where one term is used, the other should be considered as also included and recited.

A computer system (15) may be or include one or more of the following in various combinations: control systems, microcrontollers, digital signal processors (DSP), field-programmable gate arrays (FPGA), computer processors, and/or central processing units (CPU). More than one computer (or other electronic means) which together provide certain steps and/or features as described or claimed can collectively constitute a computer system (15). Various electronics within a single table may be a computer system (15) of the table.

This disclosure includes medical systems including remote controls, and remote controls for use with medical systems. The medical systems may or may not include operating tables. This disclosure also includes medical and operating tables, remote controls for use with medical and operating tables, and systems including both a medical or operating table, and also a remote control. This disclosure also includes systems with a table and a remote control, where the table controls additional devices (other than the table and the remote) at least partially based on input from the remote. This disclosure also includes methods of operating said medical systems, operating tables, and remotes both independently and collectively. This disclosure also contemplates electronic and computer executable instructions to cause the computers, remotes, and other components to execute the steps and methods described herein.

The examples described above are non-limiting examples only. It will be appreciated that the features and functions described herein can be used in various combinations.

### List of reference signs

- 11: system
- 13: medical device
- 15: computer system
- 17: remote control
- 19: screen
- 21: wireless communication interface
- 23: data
- 25: image data
- 27: displaying instructions
- 31: physical button
- 33: user input
- 35: updated image data
- 37: image
- 39: displaying instructions
- 41: rectangle
- 101: operating table
- 103: computer system
- 105: electronic device
- 107: electronic device
- 109: electronic device
- 111: connection
- 113: connection
- 115: connection
- 601: operating table
- 603: remote control
- 605: screen
- 607: button
- 609: table column
- 611: patient support surface
- 613: lower leg segment
- 615: upper leg segment
- 617: back segment
- 619: head segment

## Claims

1. A medical system comprising:
an operating table (13), the operating table (13) having a computer system (15);
a remote control (17) for remotely controlling operation of the operating table (13), the remote control (17) comprising a screen (19);
the computer system (15) and the remote control (17) being configured to communicate with each other via a communication interface (21); and
the computer system (15) being configured to provide via the communication interface (21) a graphical user interface to the remote control (17), and the remote control (17) being configured to execute the graphical user interface,
wherein the operating table (13) comprises a plurality of support sections for supporting various parts of a patient, the support sections being movable with respect to each other into different arrangements to support different patient positions of the operating table,
wherein the operating table (13) is configured to move from a first arrangement to a second arrangement by moving one or more of the support sections in response to receiving a movement signal (33) from the remote control (13), and
wherein the computer system (15) is configured to create an updated image (25) of the operating table in the second arrangement, in response to at least one of said movement signal (33) and/or the movement of the operating table from the first arrangement to the second arrangement, and to provide the updated image (25) of the operating table in the second arrangement to the remote control (13).

2. The medical system of claim 1,
wherein the computer system (15) is configured to control at least a portion of the screen (19) by the graphical user interface, when executed in the remote control (17), and to initiate the display of at least one graphical user interface element on the screen of the remote control (17).

3. The medical system of claim 2,
wherein the at least one graphical user interface element is or represents at least one of the following:
an input control element, such as a checkbox, a radio button, a dropdown list, a list box, a button, a toggle, a date field, an alphabetic keyboard, a numeric keyboard, an icon as a button, an interactive image;
a navigational component, such as a slider, an icon;
an informational component, such as a tooltip, an icon, a progress bar, a notification, a message box, a modal window, an image, a formatted text.

4. The medical system of claim 2 or 3,
wherein the at least one graphical user interface element corresponds to at least one image, and wherein the computer system (15) is configured to provide, via the communication interface, data (23) that includes image data (25) with the at least one image to the remote control (17).

5. The medical system of claim 4,
wherein the data (23) further includes displaying instructions (27) for displaying the at least one image on the screen (19); and
wherein the remote control (17) is configured, in particular via the graphical user interface, to display, in accordance with the displaying instructions (27), the at least one image on the screen (19).

6. The medical system of any one of the claims 2 to 5,
wherein the graphical user interface element corresponds to or includes instructions, in particular basic instructions, or information, such as text, and wherein the computer system (15) is configured to provide data that includes the instructions or information to the remote control (17),
wherein, optionally, the remote control (17) is configured, in particular via the graphical user interface, to display the information on the screen (19) and/or to carry out the instructions.

7. The medical system of claim 6,
wherein the instructions include an identifier for at least one image and, optionally, displaying information where to display the at least one image on the screen, wherein the remote control is configured to identify, based on the identifier, the at least one image in a database on the remote control and to display the at least one image on the screen, in particular in accordance with the displaying information.

8. The medical system of any one of the preceding claims,
wherein the computer system (25) is configured to receive user input (33) from the remote control (17) and to initiate, in accordance with the received user input (33), the display of at least one updated graphical user interface element, in particular corresponding to an updated image, on the screen (19) of the remote control (17).

9. The medical system of any one of the preceding claims,
wherein the screen (19) is a touch screen and the computer system (15) is configured to receive positional information of a user input (33) on the touch screen and to initiate the display of at least one graphical user interface element on the screen (19) in dependence on the received positional information of the user input (33).

10. The medical system of any one of the preceding claims,
wherein the at least one graphical user interface element provides, when displayed on the screen (19), an input option or a set of input options, wherein, optionally, the computer system (15) is configured to provide image data to the remote control (17) which includes at least one view of the table (13) for each input option of the set of input options, wherein, further optionally, the image data includes instructions that cause the remote control (17), in response to the user selecting an input option, to display the at least one view of the table (13) which is related to the selected input option, wherein, further optionally, different input options are provided in different regions of the screen.

11. The medical system of any one of the preceding claims,
wherein the computer system (15) is a TCP server and the remote control is a TCP client connected to the TCP server via a wireless communication interface.

12. The medical system of any one of the preceding claims,
wherein the graphical user interface provided by the computer system (15) is displayed only on a first part of the screen (19), and one or more images on a different second part of the screen (19) are provided by the remote control (17).

13. The medical system of any one of the preceding claims,
wherein the screen (19) is a touch screen, wherein the remote control (17) is configured to receive user input comprising positional information as a touch event through the touch screen,
wherein the remote control is configured to transmit the touch event to the computer system (15), and
wherein the computer system (15) is configured to receive the touch event from the remote, and to interpret the touch event as an instruction for operation of the medical system.

14. The medical system according to any one of the claims 9-13,
wherein the computer system (15) of the operating table (13) is configured to generate a list of options, in particular a list of options relating to operation of the medical system, to generate instructions for graphically presenting the list of options to the user, and to communicate the instructions to the remote control (17);
wherein the remote control (17) is configured to execute the instructions received from the computer system (15) and to display the list of options on at least part of the screen (19) of the remote control;
wherein the remote control is further configured to receive a user input associated with the list of options, the user input comprising a touch event associated with the screen (19), and then to transmit the touch event back to the computer system (15); and
wherein the computer system (15) is configured to receive and interpret the touch event as a selection from the list of options, and to act in response to the selection.

15. The medical system according to any one of the claims 9-14,
wherein the computer system (15) of the operating table (13) is configured to generate instructions for a graphical user interface element to request and receive alphanumerical input from a user, and to communicate the instructions to the remote control (17);
wherein the remote control (17) is configured to receive and execute the instructions received from the computer system (15), and to display the graphical user interface element for requesting and receiving alphanumerical input on the screen (19) of the remote control;
wherein the remote control is further configured to receive alphanumerical input from a user, the alphanumerical input comprising at least one touch event associated with the screen (19), and then to transmit the at least one touch event back to the computer system (15); and
wherein the computer system (15) is configured to receive and interpret the touch event as alphanumerical input, and to act in response to the alphanumerical input.

16. A method of operating a medical system (11) according to any of the preceding claims , the method comprising:
controlling at least a portion of a screen (19) of the remote control by a graphical user interface generated by the computer system (15), and
initiating the display of at least one graphical user interface element on the screen (19) of the remote control (17), wherein the graphical user interface of the computer system (15) is executed on the remote control (17).

17. A medical system according to any of the claims 1-15, comprising:
an operating table (101) with a computer system (103) for controlling operation of the operating table (101) or which is connected to a further computer system that controls the operation of the operating table (101), and
the computer system (103) of the operating table (101) being configured to be connected or being connected with a plurality of electronic devices (105, 107, 109) via a wireless or a wire-based connection (111, 113, 115) between each electronic device (105, 107, 109) and the computer system (103).

## Patentansprüche

1. Medizinisches System, das umfasst:
einen Operationstisch (13), wobei der Operationstisch (13) ein Computersystem (15) aufweist;
eine Fernsteuerung (17) zur Fernsteuerung eines Betriebs des Operationstischs (13), wobei die Fernsteuerung (17) einen Bildschirm (19) umfasst;
wobei das Computersystem (15) und die Fernsteuerung (17) so konfiguriert sind, dass sie über eine Kommunikationsschnittstelle (21) miteinander kommunizieren; und
wobei das Computersystem (15) konfiguriert ist, über die Kommunikationsschnittstelle (21) eine grafische Benutzeroberfläche an die Fernsteuerung (17) bereitzustellen, und wobei die Fernsteuerung (17) konfiguriert ist, die grafische Benutzeroberfläche auszuführen, wobei der Operationstisch (13) eine Mehrzahl von Stützabschnitten zum Stützen verschiedener Teile eines Patienten umfasst, wobei die Stützabschnitte relativ zueinander in verschiedene Anordnungen beweglich sind, um unterschiedliche Patientenpositionen des Operationstisches zu unterstützen, wobei der Operationstisch (13) konfiguriert ist, sich von einer ersten Anordnung in eine zweite Anordnung zu bewegen, indem einer oder mehrere der Stützabschnitte als Reaktion auf ein von der Fernsteuerung (13) empfangenes Bewegungssignal (33) bewegt werden, und wobei das Computersystem (15) konfiguriert ist, als Reaktion auf zumindest eines des Bewegungssignals (33) und/oder der Bewegung des Operationstisches von der ersten Anordnung in die zweite Anordnung ein aktualisiertes Bild (25) des Operationstisches in der zweiten Anordnung zu erzeugen, und das aktualisierte Bild (25) des Operationstisches in der zweiten Anordnung an die Fernsteuerung (13) bereitzustellen.

2. Medizinisches System nach Anspruch 1,
wobei das Computersystem (15) so konfiguriert ist, dass es mindestens einen Teil des Bildschirms (19) durch die grafische Benutzeroberfläche steuert, wenn sie in der Fernsteuerung (17) ausgeführt wird, und die Anzeige mindestens eines Elements der grafischen Benutzeroberfläche auf dem Bildschirm der Fernsteuerung (17) einleitet.

3. Medizinisches System nach Anspruch 2, wobei das mindestens eine Element der grafischen Benutzeroberfläche mindestens eines des Folgenden ist oder darstellt:
ein Eingabesteuerelement, z. B. ein Kontrollkästchen, ein Optionsfeld, eine Dropdown-Liste, ein Listenfeld, eine Schaltfläche, ein Umschalter, ein Datumsfeld, eine alphabetische Tastatur, eine numerische Tastatur, ein Symbol als Schaltfläche, ein interaktives Bild;
eine Navigationskomponente, z. B. ein Schieberegler, ein Symbol;
eine Informationskomponente, z. B. eine Kurzinfo, ein Symbol, ein Fortschrittsbalken, eine Benachrichtigung, ein Meldungsfeld, ein modales Fenster, ein Bild, ein formatierter Text.

4. Medizinisches System nach Anspruch 2 oder 3,
wobei das mindestens eine Element der grafischen Benutzeroberfläche mindestens einem Bild entspricht und wobei das Computersystem (15) so konfiguriert ist, dass es über die Kommunikationsschnittstelle Daten (23), die Bilddaten (25) mit dem mindestens einem Bild enthalten, an die Fernsteuerung (17) bereitstellt.

5. Medizinisches System nach Anspruch 4, wobei die Daten (23) ferner Anzeigeanweisungen (27) zur Anzeige des mindestens einen Bildes auf dem Bildschirm (19) beinhalten; und wobei die Fernsteuerung (17) so konfiguriert ist, dass sie insbesondere über die grafische Benutzeroberfläche gemäß den Anzeigeanweisungen (27) das mindestens eine Bild auf dem Bildschirm (19) anzeigt.

6. Medizinisches System nach einem der Ansprüche 2 bis 5, wobei das grafische Benutzeroberflächenelement Anweisungen, insbesondere grundlegenden Anweisungen, oder Informationen, wie Text, entspricht oder diese enthält, und wobei das Computersystem (15) so konfiguriert ist, dass Daten, die die Anweisungen oder Informationen enthalten, an die Fernsteuerung (17) bereitgestellt werden, wobei optional die Fernsteuerung (17) konfiguriert ist, die Informationen insbesondere über die grafische Benutzeroberfläche auf dem Bildschirm (19) anzuzeigen und/oder die Anweisungen auszuführen.

7. Medizinisches System nach Anspruch 6, wobei die Anweisungen einen Bezeichner für mindestens ein Bild und optional Anzeigeinformationen beinhalten, wo das mindestens eine Bild auf dem Bildschirm angezeigt werden soll, wobei die Fernsteuerung so konfiguriert ist, dass sie basierend auf dem Bezeichner das mindestens eine Bild in einer Datenbank auf der Fernsteuerung identifiziert und das mindestens eine Bild auf dem Bildschirm anzeigt, insbesondere gemäß den Anzeigeinformationen.

8. Medizinisches System nach einem der vorhergehenden Ansprüche, wobei das Computersystem (25) konfiguriert ist, eine Benutzereingabe (33) von der Fernsteuerung (17) zu empfangen und gemäß der empfangenen Benutzereingabe (33) die Anzeige von mindestens einem aktualisierten Element der grafischen Benutzeroberfläche insbesondere entsprechend einem aktualisierten Bild auf dem Bildschirm (19) der Fernsteuerung (17) einzuleiten.

9. Medizinisches System nach einem der vorhergehenden Ansprüche, wobei der Bildschirm (19) ein Touchscreen ist und das Computersystem (15) so konfiguriert ist, dass es Positionsinformationen einer Benutzereingabe (33) auf dem Touchscreen empfängt und die Anzeige mindestens eines Elements der grafischen Benutzeroberfläche auf dem Bildschirm (19) in Abhängigkeit von den empfangenen Positionsinformationen der Benutzereingabe (33) einleitet.

10. Medizinisches System nach einem der vorhergehenden Ansprüche, wobei das mindestens eine Element der grafischen Benutzeroberfläche, wenn es auf dem Bildschirm (19) angezeigt wird, eine Eingabeoption oder einen Satz von Eingabeoptionen bereitstellt, wobei das Computersystem (15) optional konfiguriert ist, Bilddaten an die Fernsteuerung (17) bereitzustellen, die für jede Eingabeoption des Satzes von Eingabeoptionen mindestens eine Ansicht des Tisches (13) beinhalten, wobei die Bilddaten ferner optional Anweisungen enthalten, die die Fernsteuerung (17) veranlassen, als Reaktion auf die Auswahl einer Eingabeoption durch den Benutzer die mindestens eine Ansicht des Tisches (13) anzuzeigen, die sich auf die ausgewählte Eingabeoption bezieht, wobei ferner optional unterschiedliche Eingabeoptionen in verschiedenen Bereichen des Bildschirms bereitgestellt werden.

11. Medizinisches System nach einem der vorhergehenden Ansprüche, wobei das Computersystem (15) ein TCP-Server ist und die Fernsteuerung ein TCP-Client ist, der über eine drahtlose Kommunikationsschnittstelle mit dem TCP-Server verbunden ist.

12. Medizinisches System nach einem der vorhergehenden Ansprüche, wobei die vom Computersystem (15) bereitgestellte grafische Benutzeroberfläche nur auf einem ersten Teil des Bildschirms (19) angezeigt wird und ein oder mehrere Bilder auf einem anderen zweiten Teil des Bildschirms (19) durch die Fernsteuerung (17) bereitgestellt werden.

13. Medizinisches System nach einem der vorhergehenden Ansprüche, wobei der Bildschirm (19) ein Touchscreen ist, wobei die Fernsteuerung (17) konfiguriert ist, Benutzereingaben zu empfangen, die Positionsinformationen als Berührungsereignis durch den Touchscreen umfassen, wobei die Fernsteuerung so konfiguriert ist, dass sie das Berührungsereignis an das Computersystem (15) übermittelt, und wobei das Computersystem (15) konfiguriert ist, das Berührungsereignis von der Fernsteuerung zu empfangen und das Berührungsereignis als eine Anweisung zum Betrieb des medizinischen Systems zu interpretieren.

14. Medizinisches System nach einem der Ansprüche 9 bis 13, wobei das Computersystem (15) des Operationstisches (13) so konfiguriert ist, eine Liste von Optionen, insbesondere eine Liste von Optionen, die sich auf den Betrieb des medizinischen Systems beziehen, zu generieren, um Anweisungen zur grafischen Darstellung der Liste der Optionen für den Benutzer zu generieren, und die Anweisungen an die Fernsteuerung (17) zu übermitteln;
wobei die Fernsteuerung (17) so konfiguriert ist, dass sie die vom Computersystem (15) empfangenen Anweisungen ausführt und die Liste von Optionen auf zumindest einem Teil des Bildschirms (19) der Fernsteuerung anzeigt;
wobei die Fernsteuerung ferner so konfiguriert ist, dass sie eine Benutzereingabe empfängt, die der Liste von Optionen zugeordnet ist, wobei die Benutzereingabe ein Berührungsereignis umfasst, das dem Bildschirm (19) zugeordnet ist, und dann das Berührungsereignis zurück an das Computersystem (15) übermittelt; und wobei das Computersystem (15) so konfiguriert ist, dass es das Berührungsereignis als Auswahl aus der Liste von Optionen empfängt und interpretiert und als Reaktion auf die Auswahl handelt.

15. Medizinisches System nach einem der Ansprüche 9 bis 14, wobei das Computersystem (15) des Operationstisches (13) konfiguriert ist, Anweisungen für ein Element der grafischen Benutzeroberfläche zu generieren, um alphanumerische Eingaben von einem Benutzer anzufordern und zu empfangen und die Anweisungen an die Fernsteuerung (17) zu übermitteln;
wobei die Fernsteuerung (17) so konfiguriert ist, dass sie die vom Computersystem (15) empfangenen Anweisungen empfängt und ausführt und das Element der grafischen Benutzeroberfläche zum Anfordern und Empfangen einer alphanumerischen Eingabe auf dem Bildschirm (19) der Fernsteuerung anzeigt;
wobei die Fernsteuerung ferner so konfiguriert ist, dass sie eine alphanumerische Eingabe von einem Benutzer empfängt, wobei die alphanumerische Eingabe mindestens ein Berührungsereignis umfasst, das dem Bildschirm (19) zugeordnet ist, und dann das mindestens eine Berührungsereignis zurück an das Computersystem (15) übermittelt; und wobei das Computersystem (15) konfiguriert ist, das Berührungsereignis als alphanumerische Eingabe zu empfangen und zu interpretieren und als Reaktion auf die alphanumerischen Eingabe zu handeln.

16. Verfahren zum Betreiben eines medizinischen Systems (11) nach einem der vorhergehenden Ansprüche, wobei das Verfahren umfasst:
Steuern mindestens eines Teils eines Bildschirms (19) der Fernsteuerung über eine grafische Benutzeroberfläche, die vom Computersystem (15) erzeugt wird, und
Einleiten der Anzeige von mindestens einem Element der grafischen Benutzeroberfläche auf dem Bildschirm (19) der Fernsteuerung (17), wobei die grafische Benutzeroberfläche des Computersystems (15) auf der Fernsteuerung (17) ausgeführt wird.

17. Medizinisches System nach einem der Ansprüche 1 bis 15, das umfasst: einen Operationstisch (101) mit einem Computersystem (103) zur Steuerung eines Betriebs des Operationstisches (101) oder das mit einem weiteren Computersystem verbunden ist, das den Betrieb des Operationstisches (101) steuert, und
wobei das Computersystem (103) des Operationstisches (101) so konfiguriert ist, dass es über eine drahtlose oder drahtgebundene Verbindung (111, 113, 115) zwischen jedem elektronischen Gerät (105, 107, 109) und dem Computersystem (103) mit einer Mehrzahl von elektronischen Geräten (105, 107, 109) verbunden wird, oder damit verbunden ist.

## Revendications

1. Système médical comprenant :
une table d'opération (13), la table d'opération (13) ayant un système informatique (15) ;
une télécommande (17) pour commander à distance le fonctionnement de la table d'opération (13), la télécommande (17) comprenant un écran (19) ;
le système informatique (15) et la télécommande (17) étant configurés pour communiquer entre eux via une interface de communication (21) ; et
le système informatique (15) est configuré pour fournir, via l'interface de communication (21), une interface utilisateur graphique à la télécommande (17), et la télécommande (17) est configurée pour exécuter l'interface utilisateur graphique,
dans lequel la table d'opération (13) comprend une pluralité de sections de support destinées à supporter différentes parties d'un patient, les sections de support étant mobiles les unes par rapport aux autres et peuvent être disposées de différentes manières afin de supporter différentes positions du patient sur la table d'opération,
dans lequel la table d'opération (13) est configurée pour passer d'une première configuration à une deuxième configuration en déplaçant une ou plusieurs des sections de support en réponse à la réception d'un signal de mouvement (33) provenant de la télécommande (13), et
dans lequel le système informatique (15) est configuré pour créer une image mise à jour (25) de la table d'opération dans la deuxième configuration, en réponse à au moins un des signaux de mouvement (33) et/ou au déplacement de la table d'opération de la première configuration à la deuxième configuration, et pour fournir l'image mise à jour (25) de la table d'opération dans la deuxième configuration à la télécommande (13).

2. Système médical selon la revendication 1,
dans lequel le système informatique (15) est configuré pour commander au moins une partie de l'écran (19) par l'interface utilisateur graphique, lorsqu'il est exécuté dans la télécommande (17), et pour lancer l'affichage d'au moins un élément d'interface utilisateur graphique sur l'écran de la télécommande (17).

3. Système médical selon la revendication 2,
dans lequel l'au moins un élément d'interface utilisateur graphique est ou représente au moins l'un des éléments suivants :
un élément de commande d'entrée, tel qu'une case à cocher, un bouton radio, une liste déroulante, une zone de liste, un bouton, un bouton bascule, un champ de date, un clavier alphabétique, un clavier numérique, une icône en tant que bouton, une image interactive ;
un élément de navigation, tel qu'un curseur, une icône ;
un élément d'information, tel qu'une infobulle, une icône, une barre de progression, une notification, une boîte de message, une fenêtre modale, une image, un texte formaté.

4. Système médical selon la revendication 2 ou 3,
dans lequel au moins un élément d'interface utilisateur graphique correspond à au moins une image, et dans lequel le système informatique (15) est configuré pour fournir, via l'interface de communication, des données (23) qui incluent des données d'image (25) avec au moins une image à la télécommande (17) .

5. Système médical selon la revendication 4,
dans lequel les données (23) comprennent en outre des instructions d'affichage (27) pour afficher l'au moins une image sur l'écran (19) ; et
dans lequel la télécommande (17) est configurée, notamment via l'interface utilisateur graphique, pour afficher, conformément aux instructions d'affichage (27), l'au moins une image sur l'écran (19).

6. Système médical selon l'une quelconque des revendications 2 à 5,
dans lequel l'élément d'interface utilisateur graphique correspond à ou comprend des instructions, en particulier des instructions de base, ou des informations, telles que du texte, et dans lequel le système informatique (15) est configuré pour fournir des données qui comprennent les instructions ou les informations à la télécommande (17),
dans lequel, éventuellement, la télécommande (17) est configurée, notamment via l'interface utilisateur graphique, pour afficher les informations sur l'écran (19) et/ou pour exécuter les instructions.

7. Système médical selon la revendication 6,
dans lequel les instructions comprennent un identifiant pour au moins une image et, éventuellement, des informations d'affichage indiquant où afficher l'au moins une image sur l'écran, dans lequel la télécommande est configurée pour identifier, sur la base de l'identifiant, l'au moins une image dans une base de données sur la télécommande et pour afficher l'au moins une image sur l'écran, notamment conformément aux informations d'affichage.

8. Système médical selon l'une quelconque des revendications précédentes,
dans lequel le système informatique (25) est configuré pour recevoir une entrée utilisateur (33) de la télécommande (17) et pour initier, conformément à l'entrée utilisateur reçue (33), l'affichage d'au moins un élément d'interface utilisateur graphique mis à jour, correspondant notamment à une image mise à jour, sur l'écran (19) de la télécommande (17).

9. Système médical selon l'une quelconque des revendications précédentes,
dans lequel l'écran (19) est un écran tactile et le système informatique (15) est configuré pour recevoir des informations de position d'une entrée utilisateur (33) sur l'écran tactile et pour lancer l'affichage d'au moins un élément d'interface utilisateur graphique sur l'écran (19) en fonction des informations de position reçues de l'entrée utilisateur (33).

10. Système médical selon l'une quelconque des revendications précédentes,
dans lequel l'au moins un élément d'interface utilisateur graphique fournit, lorsqu'il est affiché à l'écran (19), une option d'entrée ou un ensemble d'options d'entrée, dans lequel, éventuellement, le système informatique (15) est configuré pour fournir des données d'image à la télécommande (17) qui comprennent au moins une vue de la table (13) pour chaque option d'entrée de l'ensemble d'options d'entrée, dans lequel, en outre, éventuellement, les données d'image comprennent des instructions qui amènent la télécommande (17), en réponse à la sélection d'une option d'entrée par l'utilisateur, à afficher l'au moins une vue de la table (13) liée à l'option d'entrée sélectionnée, dans lequel, en outre, éventuellement, différentes options d'entrée sont fournies dans différentes régions de l'écran.

11. Système médical selon l'une quelconque des revendications précédentes,
dans lequel le système informatique (15) est un serveur TCP et la télécommande est un client TCP connecté au serveur TCP via une interface de communication sans fil.

12. Système médical selon l'une quelconque des revendications précédentes,
dans lequel l'interface utilisateur graphique fournie par le système informatique (15) est affichée uniquement sur une première partie de l'écran (19), et une ou plusieurs images sur une seconde partie différente de l'écran (19) sont fournies par la télécommande (17).

13. Système médical selon l'une quelconque des revendications précédentes,
dans lequel l'écran (19) est un écran tactile, dans lequel la télécommande (17) est configurée pour recevoir une entrée utilisateur comprenant des informations de position sous forme d'événement tactile via l'écran tactile,
dans lequel la télécommande est configurée pour transmettre l'événement tactile au système informatique (15), et
dans lequel le système informatique (15) est configuré pour recevoir l'événement tactile de la télécommande et pour interpréter l'événement tactile comme une instruction de fonctionnement du système médical.

14. Système médical selon l'une quelconque des revendications 9 à 13,
dans lequel le système informatique (15) de la table d'opération (13) est configuré pour générer une liste d'options, en particulier une liste d'options relatives au fonctionnement du système médical, pour générer des instructions pour présenter graphiquement la liste d'options à l'utilisateur et pour communiquer les instructions à la télécommande (17) ;
dans lequel la télécommande (17) est configurée pour exécuter les instructions reçues du système informatique (15) et pour afficher la liste des options sur au moins une partie de l'écran (19) de la télécommande ;
dans lequel la télécommande est en outre configurée pour recevoir une entrée utilisateur associée à la liste d'options, l'entrée utilisateur comprenant un événement tactile associé à l'écran (19), puis pour transmettre l'événement tactile au système informatique (15) ; et
dans lequel le système informatique (15) est configuré pour recevoir et interpréter l'événement tactile comme une sélection dans la liste d'options, et pour agir en réponse à la sélection.

15. Système médical selon l'une quelconque des revendications 9 à 14, dans lequel le système informatique (15) de la table d'opération (13) est configuré pour générer des instructions pour un élément d'interface utilisateur graphique afin de demander et de recevoir une entrée alphanumérique d'un utilisateur, et de communiquer les instructions à la télécommande (17) ;
dans lequel la télécommande (17) est configurée pour recevoir et exécuter les instructions reçues du système informatique (15), et pour afficher l'élément d'interface utilisateur graphique pour demander et recevoir une entrée alphanumérique sur l'écran (19) de la télécommande ;
dans lequel la télécommande est en outre configurée pour recevoir une entrée alphanumérique d'un utilisateur, l'entrée alphanumérique comprenant au moins un événement tactile associé à l'écran (19), puis pour transmettre l'au moins un événement tactile au système informatique (15) ; et
dans lequel le système informatique (15) est configuré pour recevoir et interpréter l'événement tactile comme une entrée alphanumérique, et pour agir en réponse à l'entrée alphanumérique.

16. Procédé de fonctionnement d'un système médical (11) selon l'une quelconque des revendications précédentes, le procédé comprenant :
la commande d'au moins une partie d'un écran (19) de la télécommande par une interface utilisateur graphique générée par le système informatique (15), et
l'initiation de l'affichage d'au moins un élément d'interface utilisateur graphique sur l'écran (19) de la télécommande (17), dans lequel l'interface utilisateur graphique du système informatique (15) est exécutée sur la télécommande (17).

17. Système médical selon l'une quelconque des revendications 1 à 15, comprenant :
une table d'opération (101) avec un système informatique (103) pour commander le fonctionnement de la table d'opération (101) ou qui est connecté à un autre système informatique qui commande le fonctionnement de la table d'opération (101), et le système informatique (103) de la table d'opération (101) étant configuré pour être connecté ou étant connecté à une pluralité de dispositifs électroniques (105, 107, 109) via une connexion sans fil ou filaire (111, 113, 115) entre chaque dispositif électronique (105, 107, 109) et le système informatique (103).
